# EUROPEAN PATENT APPLICATION

(11) **EP 1 792 574 A1**
(43) Date of publication of application: **06.06.2007**
(21) Application number: 05780232.4
(22) Date of filing: 15.08.2005
(51) Int. Cl.: A61B 10/00, A61B 1/00

(54) **INSTRUMENT FOR COLLECTING VITAL TISSUE, ENDOSCOPE SYSTEM AND METHOD OF COLLECTING VITAL TISSUE**

(30) Priority: 14.09.2004 JP 2004267073
(71) Applicant: Olympus Corporation, Tokyo 151-0072 (JP)
(72) Inventor: KONDO, Seiji c/o Intellectual Property Support Department, 2-3, Kuboyama-cho, Hachioji-shi Tokyo 192-8512 (JP); SANUKI, Hiromi c/o Intellectual Property Support Department, 2-3, Kuboyama-cho, Hachioji-shi Tokyo 192-8512 (JP)
(74) Representative: von Hellfeld, Axel
(86) International application number: PCT/JP2005/014928
(87) International publication number: WO 2006/030596

(57) **Abstract**

A living-tissue sampling instrument includes a hollow tubular member having a sampling part configured to sample living tissues at a distal end of the tubular member, and a suction mechanism configured to draw the living tissue sampled by the sampling part at a proximal end of the tubular member. Projections extending inwards are provided on least one part of the inner circumferential surface of the tubular member.

## Description

### Technical Field

The present invention relates to a living-tissue sampling living-tissue sampling instrument in a percutaneous manner by using an endoscope, to an endoscope system, and to a method of sampling living tissues.

### Background Art

Generally, living tissues are sampled and subjected to pathological examination, biochemical analysis, genomic analysis, or the like. Living tissues are sampled with such instrument as are disclosed in, for example, Jpn. Pat. Appln. KOKAI Publication No. 2001-275947 and PCT National Publication No. 2000-516832.

As disclosed in Jpn. Pat. Appln. KOKAI Publication No. 2001-275947, a suction tube having, at the distal end, a hollow puncture needle for use with endoscopes, is inserted into a body cavity through the instrument-insertion channel of an endoscope. The target tissue is pierced with the puncture needle, while being observing the tissue through the endoscope. The tissue thus pierced is drawn and sampled. The living tissue thus sampled by using the puncture needle for use with endoscopes is transported from the endoscopic examination room to another examination room. In the other examination room, the tissue undergoes pathological examination, biochemical analysis, genomic analysis, or the like.

PCT National Publication No. 2000-516832 discloses a biopsy-forceps instrument that is used with endoscopes. This biopsy-forceps instrument has a forceps part that is arranged at the distal end of the suction tube. Thus, the living tissue sampled with the forceps part protruding from the distal end of the instrument-insertion channel of the endoscope can be drawn from the distal end of the suction tube toward the proximal end thereof.

Jpn. Pat. Appln. KOKAI Publication No. 2001-124767 discloses a method in which the flow passage of a syringe having a bent part is moved back and forth, thereby to destroy the cell membranes of a living tissue. Once the cell membranes have been destroyed, the chromosomes can be sampled with ease.

With the instrument disclosed in Jpn. Pat. Appln. KOKAI Publication No. 2001-275947 and PCT National Publication No. 2000-516832, it takes much time to perform the next treatment after a living sample is obtained. Particularly, much time is required before the living tissue is destroyed to extract the nucleic acid or before the nucleic acid extracted is stabilized. The living tissue sampled may therefore deteriorate. In this case, it difficult to make an accurate diagnosis. Further, to treat the living tissue with a medicine, the tissue must be transported into a vessel. While being so transported, the living tissue may be contaminated or may contaminate the environment.

Since the living tissue is sampled during an operation, the time, manpower and space available for processing the sampled tissue are much limited. Hence, it is desired that the living tissue sampled be easily and quickly destroyed, thereby to extract and stabilize the nucleic acid.

The instrument disclosed in Jpn. Pat. Appln. KOKAI Publication No. 2001-124767 has a bent part at a syringe. To guide the instrument through the instrument-insertion channel of an endoscope, the channel must therefore have a large diameter in compliance with the instrument-insertion distance the bent part. Hence, it may be difficult to sample living tissues through the endoscope, by using this instrument, depending on the diameter of the channel of the endoscope.

### Disclosure of Invention

This invention has been made to solve this problem. An object of the invention is to provide a living-tissue sampling instrument, an endoscope system, and a method of sampling living tissues, which can easily and quickly destroy sampled living tissues, thereby to shorten the time required to prepare for pathological examination, biochemical analysis, genomic analysis, or the like.

A living-tissue sampling instrument according to one aspect of the present invention includes:
a hollow tubular member which has a distal end portion having a sampling part to sample living tissue, and a distal end portion having a suction mechanism configured to draw living tissue sampled by the sampling part,
wherein the tubular member has projections extending inwards, on least one part of an inner circumferential surface.

In the instrument so configured, the living tissue sampled by the sampling part collides with the projections as it is drawn through the tubular member. Thus, the living tissue sampled can be easily and quickly destroyed. This can shorten the time required to prepare for pathological examination, biochemical analysis, genomic analysis, or the like.

The projections may preferably be provided at random in the tubular member.

Since the projections are provided at random on the inner circumferential surface of the tubular member and have random sizes. Therefore, the living tissue can be reliably destroyed as it moves from the distal end of the tubular member toward the proximal end thereof.

The tubular member may preferably include an inner diameter that is unevenness because of the projections.

Therefore, the tubular member may be deformed in terms of inner diameter and outer diameter, or only the inner circumferential surface of the tubular member may be deformed.

Preferably, the projections may be formed independently of one another.

Then, the projections can be easily formed at random positions in random sizes.

Further, at least some of the projections may preferably be continuous to one another.

Thus, the projections may be formed to define, for example, a female screw, and are therefore easy to form.

Preferably, the projections should be formed by applying a pressure on the tubular member from outside.

The tubular member can therefore be formed easily.

The suction mechanism may preferably include a suction cavity which is able to accumulate the living tissue sampled by the sampling part.

The living tissue destroyed as it is drawn by suction can be accumulated, without using another container.

A solution to be mixed with the living tissue sampled by the sampling part may preferably be accumulated in the suction cavity.

In this case, the living tissue destroyed can be mixed with the solution. This can retard the deterioration of the living tissue.

Preferably, a container capable of storing the living tissue sampled by the sampling part may be removably provided between a proximal end of the tubular member and the suction cavity.

Thus, the living tissue destroyed as it is drawn by suction can be accumulated in this container. Once the container has been removed, it is easy to transport the living tissue to a site where the tissue undergoes, for example, pathological examination, biochemical analysis, genomic analysis, or the like.

Preferably, the container may accumulate a solution to be mixed with the living tissue sampled by the sampling part.

The living tissue destroyed can be mixed with the solution, whereby the deterioration of the living tissue can be retarded. Further, the solution can be used as a regent.

Preferably, the suction mechanism may include a valve mechanism provided between a proximal end of the tubular member and the suction cavity, the valve mechanism being able to be switched between a communicating state in which to communicate the sampling part to the suction cavity, and a cutoff state in which to cut off the sampling part to the suction cavity, the valve mechanism including a passage that communicates with the suction cavity in the cutoff state.

Therefore, after the living tissue destroyed can be expelled when the valve mechanism is operated, after the tissue has been temporarily accumulated in the suction cavity.

Preferably, a container capable of storing the living tissue sampled by the sampling part may be arranged at an end of the passage of the valve mechanism.

This container can store the living tissue expelled from the tubular member. In addition, once the container has been removed, it is easy to transport the living tissue to a site where the tissue undergoes, for example, pathological examination, biochemical analysis, genomic analysis, or the like.

A passage-switching mechanism may preferably includes a plurality of communication passages to be selectively connected to the passage, and is arranged to being able to connect and disconnect containers capable of storing the living tissue to and from the communication passages, respectively

In this case, the living tissue temporarily accumulated in the suction cavity can be divided into parts. Further, living tissues sampled at various positions can be expelled into different containers.

Preferably, at least one of the tubular member and the suction mechanism may include a filter configured to capture a part of the living tissue sampled by the sampling part.

The filter may be impregnated with, for example, an RNA-stabilizing agent. In this case, the nucleic acid in the cells captured can be stabilized immediately after the cells have been captured.

The filter may preferably include a bio-compatibility compound to living tissues on one side facing the sampling part.

Specific cells or the like in the living tissue can therefore be adsorbed to the filter as the living tissue is drawn by suction.

Preferably, at least one of the tubular member and the suction mechanism may include:
magnetic beads each having, on outer surface, bio-compatibility compound to the living tissues; and
one of a magnet and an electromagnet, which applies magnetic force to the magnetic beads.

Specific cells or the like in the living tissue are therefore adsorbed to the magnetic beads when the living tissue is drawn by suction.

The bio-compatibility compound to the living tissues may preferably be a cancer-cell recognizing antibody.

Then, the cancer cells are left at the filter or on the magnetic beads, while the other cells are accumulated in, for example, the suction cavity, when the living tissue is drawn by suction.

Preferably, a cooling mechanism configured to cool the living tissue sampled by the sampling part may be connected to the suction mechanism.

The deterioration of the living tissue destroyed can therefore be prevented.

The sampling part may preferably include a hollow puncture needle to be thrust into living tissues.

Once the puncture needle has been thrust into the living tissue, the tissue can be destroyed as it passes through the tubular member.

Preferably, the tubular member may be rigid at an outer circumferential surface.

This makes it easy to sample the living tissue in, for example, a percutaneous manner.

Preferably, the tubular member may include flexibility.

This makes it easy to sample the living tissue in, for example, a percutaneous manner.

The sampling part may preferably include a forceps able to be opened and closed to hold living tissues.

The living tissue sampled by using the forceps can therefore be destroyed as it is drawn by suction. Moreover, any desired living tissue can be more reliably sampled by using the forceps in combination with a puncture needle.

An endoscope system according to an aspect of this invention includes an endoscope and a living-tissue sampling instrument. The endoscope includes an elongated insertion section which has an instrument-insertion channel extending in an axial direction, and an operation section which is arranged at a proximal end of the insertion section. The living-tissue sampling instrument includes a hollow tubular member having such an outer diameter that the member is inserted into and pulled from the instrument-insertion channel, having projections extending inwards, on least one part of an inner circumferential surface, and being longer than the instrument-insertion channel, a sampling part provided at a distal end of the tubular member, able of be inserted and configured to sample living tissues, and a suction mechanism provided at a proximal end of the tubular member and configured to draw, by suction, a living tissue sampled by the sampling part.

In the system thus configured, the endoscope and the living-tissue sampling instrument can be used in combination. Living tissues can be destroyed as they are sampled and drawn by suction by means of the living-tissue sampling instrument.

The suction mechanism may preferably include a container capable of storing the living tissue sampled by the sampling part and accumulate the solution, and a suction part able to be switched between a communicating state in which to communicate with the container and a cutoff state in which to be disconnected from the container.

Hence, the living tissue destroyed in the suction part can be accumulated in the container as the suction part draws the tissue by suction. At this time, the suction part can be switched from the communicating state to the cutoff state, or vice versa. The container can therefore be attached to and removed from the suction part. Since the container contains a solution, the living tissue destroyed can be mixed with the solution. The deterioration of the tissue is thereby retarded. Further, the solution can be used as a reagent.

Preferably, the suction mechanism may include:
a suction cavity which is able to accumulate the living tissue sampled by the sampling part; and
a valve mechanism which is provided between a proximal end of the tubular member and the suction cavity, the valve mechanism being able to be switched between a communicating state in which to communicate the sampling part to the suction cavity, and a cutoff state in which to cut off the sampling part to the suction cavity, the valve mechanism including a passage that communicates with the suction cavity in the cutoff state.

The living tissue destroyed by suction can therefore be accumulated, without using any other container. The tissue destroyed can be first temporarily be accumulated in the suction cavity and then expelled through the passage communicating with the suction cavity as the valve mechanism is operated.

The sampling part may preferably include a hollow puncture needle to be thrust into living tissues.

With the hollow puncture needle thrust in the living tissue, the tissue can be destroyed as it is drawn by suction through the needle and the tubular member. Thus, the living tissue can be easily sampled by using an endoscope.

Preferably, the sampling part may include a tube which is able to be inserted into a duct in a living body.

Thus, if the tube is inserted into a duct in the living body (for example, the pancreatic duct or the bile duct), any secretory liquid can be sampled from the duct and passed through a filter or the like, which filters out the exfoliated tissue or cells. The tissue or cells thus sampled can be subjected to an examination.

The sampling part may preferably include a forceps able to be opened and closed to hold living tissues, and an opening/closing mechanism is provided at a proximal end of the tubular member and is configured to open and close the forceps.

Thus, the living tissue can be destroyed as it is drawn by suction when the opening/closing mechanism is held and operated, thereby manipulating the forceps. In addition, the desired living tissue can be more reliably sampled, by using the forceps in combination with the puncture needle.

A method of sampling living tissues, according to an aspect of the present invention, uses a living-tissue sampling instrument. The method includes:
drawing the living tissue sampled by the sampling part and making the living tissue collide with the projections provided in the tubular member, thereby destroying the living tissue; and
accumulating, in the suction mechanism, the living tissue made to collide with the projections and thereby destroyed.

The living tissue sampled and destroyed can therefore be accumulated in the suction mechanism. This holds true of the case where the living tissue is sampled in a percutaneous manner.

A method of sampling living tissues, according to another aspect of this invention, uses a living-tissue sampling instrument. This method includes:
drawing the living tissue sampled by the sampling part and making the living tissue collide with the projections provided in the tubular member, thereby destroying the living tissue;
accumulating, in the container, the living tissue destroyed; and
removing the container from a junction between the proximal end of the tubular member and the suction mechanism.

The living tissue sampled and destroyed can therefore be accumulated in the suction mechanism. Once the container has been removed from the living-tissue sampling instrument, the living tissue destroyed can be easily analyzed. This holds true of the case where the living tissue is sampled in a percutaneous manner.

A method of sampling living tissues, according to still another aspect of this invention, uses a living-tissue sampling instrument. The method includes:
drawing the living tissue sampled by the sampling part and making the living tissue collide with the projections provided in the tubular member, thereby destroying the living tissue;
temporarily accumulating, in the container, the living tissue destroyed;
switching the valve mechanism, thereby setting the suction mechanism and an interior of the container in the communicating state;
expelling the living tissue from the suction mechanism into the container through the valve mechanism; and
detaching the container.

The living tissue sampled and destroyed can therefore be accumulated in the suction mechanism. While the tissue is being so accumulated, the valve mechanism is switched, setting the suction mechanism and the container in a cut-off state. Then, the tissue can be easily expelled into the container through the valve mechanism. This holds true of the case where the living tissue is sampled in a percutaneous manner.

The drawing the living tissue sampled by the sampling part and making the living tissue collide with the projections provided in the tubular member, thereby destroying the living tissue may preferably include projecting the sampling part from a distal end of a instrument-insertion channel of an endoscope.

Therefore, the living tissue can be sampled in a percutaneous manner.

### Brief Description of Drawings

FIG. 1 is a schematic perspective view showing an endoscope system according to a first embodiment of the present invention;
FIG. 2 is a schematic perspective view showing a living-tissue sampling instrument for use in the endoscope system according to the first embodiment;
FIG. 3 is a schematic perspective view showing the needle-shaped sampling part for sampling living tissues and tube distal end of the living-tissue sampling instrument, according to the first embodiment;
FIG. 4 is a schematic longitudinal sectional view of the tube of the living-tissue sampling instrument, according to the first embodiment;
FIG. 5A is a schematic sectional view showing the forceps-shaped sampling part of the living-tissue sampling instrument, according to the first embodiment;
FIG. 5B is also a schematic sectional view showing the forceps-shaped sampling part of the living-tissue sampling instrument, according to the first embodiment;
FIG. 6 is a schematic view showing a living-tissue sampling instrument, according to a second embodiment;
FIG. 7 is a schematic view showing the proximal end of the tube and suction mechanism of a living-tissue sampling instrument, according to a third embodiment;
FIG. 8 is a schematic view showing the proximal end of the tube and suction mechanism of a living-tissue sampling instrument, according to a fourth embodiment;
FIG. 9A is a schematic view showing the proximal end of the tube and suction mechanism of a living-tissue sampling instrument, according to a fifth embodiment;
FIG. 9B is a schematic view showing the valve mechanism arranged at the proximal end of the tube of the living-tissue sampling instrument, according to the fifth embodiment;
FIG. 10 is a schematic view showing the proximal end of the tube and suction mechanism of a living-tissue sampling instrument, according to a sixth embodiment;
FIG. 11 is a schematic view showing the proximal end of the tube and suction mechanism of a living-tissue sampling instrument, according to a seventh embodiment;
FIG. 12 is a schematic view showing the proximal end of the tube and suction mechanism of a living-tissue sampling instrument, according to an eighth embodiment;
FIG. 13 is a schematic view showing the proximal end of the tube and suction mechanism of a living-tissue sampling instrument, according to a ninth embodiment;
FIG. 14 is a schematic view showing the proximal end of the tube and suction mechanism of a living-tissue sampling instrument, according to a tenth embodiment;
FIG. 15A is a schematic longitudinal section view showing a continuous projection provided in the tube of a living-tissue sampling instrument, according to an eleventh embodiment; and
FIG. 15B is a schematic longitudinal section view showing the tube of a living-tissue sampling instrument, according to an eleventh embodiment, the tube having been squeezed and thus having projections and depressions.

### Best Mode for Carrying Out the Invention

Best modes for carrying out the present invention (hereinafter referred to as embodiments) will be described, with reference to the accompanying drawings.

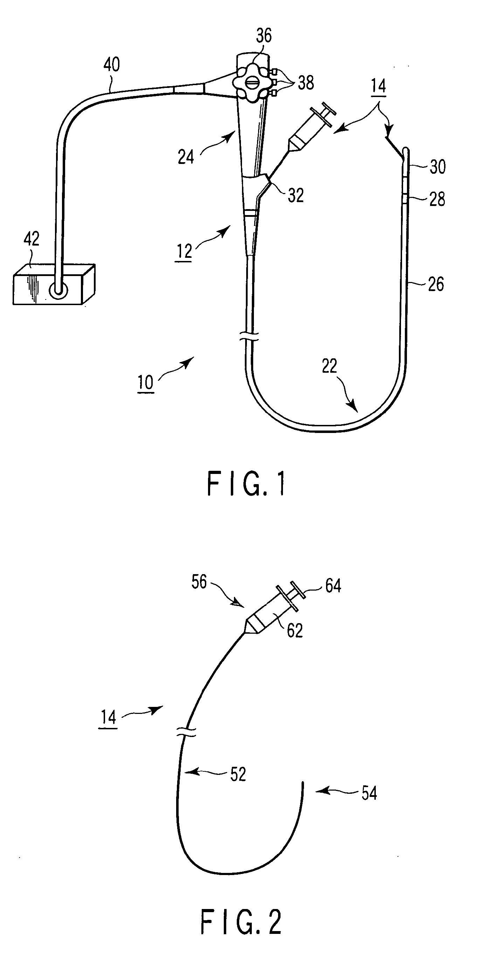

The first embodiment will be described with reference to FIGS. 1 to 5B.

As FIG. 1 shows, an endoscope system 10 according to the embodiment includes an endoscope 12 and a living-tissue sampling instrument 14 for use in combination with the endoscope 12. The term "living tissues" used in conjunction with the embodiment include, in addition to ordinary ones, tumors, cysts and exfoliated tissues contained in the liquids (e.g., liquid in the abdominal cavity, liquid in the thoracic cavity, pancreatic juice, bile, etc.) and cells.

The endoscope 12 includes an insertion section 22 and an operation section 24. The insertion section 22 is a long tubular member than can be inserted into body cavities. The operation section 24 is provided at the proximal end of the insertion section 22 and can be held and operated by an operator.

The insertion section 22 includes a flexible tube part 26 having flexibility, a bending part 28 that can be bent, and a distal-end rigid part 30. The proximal end of the flexible tube part 26 is coupled with the operation section 24. The bending part 28 is provided at the distal end of the flexible tube part 26. The distal-end rigid part 30 is arranged at the distal end of the bending part 28.

In the distal-end rigid part 30, an illumination optical system (not shown) and an objective optical system (not shown, either) are arranged side by side. The illumination optical system applies to an object of observation the illumination light guided from the light source provided in a controller 42, which will be described later. The objective optical system receives an optical image of the object illuminated with the illumination light and transmits the optical image to the controller 42.

The illumination optical system emits the illumination light in various directions different with respect to the longitudinal direction of the insertion section 22. The objective optical system has an optical axis aligned with the direction of the illumination light, to receive the image of any object that is illuminated with the illumination light. That is, the illumination optical system and the objective optical system enable the operator to observe anything in different direction with respect to the longitudinal axis of the insertion section 22. The illumination optical system and the objective optical system are of so-called oblique-viewing type or so-called side-viewing type. In this embodiment, the endoscope 12 is of the side-viewing type. Nonetheless, it does not matter whether it is of the oblique-viewing type or the so-called direct-viewing type.

The insertion section 22 includes an instrument-insertion channel (not shown). The instrument-insertion channel is arranged parallel to the illumination optical system and objective optical system. At the distal end of the instrument-insertion channel, a hebel (not shown) is arranged to hold an elongated instrument that may be inserted through the instrument-insertion channel. Hence, the tube 52 of the living-tissue sampling instrument 14, which will be described later, can be held and fixed at a desired position. A forceps valve 32 is arranged at the proximal end of the instrument-insertion channel. The forceps valve 32 therefore lies at the proximal end of the insertion section 22.

An angulation wire (not shown) is coupled, at distal end, to the bending part 28. The angulation wire extends through the flexible tube part 26 and is coupled, at the proximal end, to an angulation knob 36 provided on the operation section 24. The knob 36 will be described later.

The operation section 24 includes switches 38, in addition to the angulation knob 36. When operated, the angulation knob 36 bends the bending part 28 of the insertion section 22. The switches 38 include an air supply switch, a water supply switch and the like. When the air supply switch and water supply switch are operated, air and water are supplied from the distal end of the insertion section 22. A universal cable 40 is secured, at one end, to the operation section 24. At the other end of the universal cable 40, the controller 42 is arranged. The controller 42 incorporates a light source that applies light toward the illumination optical system provided in the distal-end rigid part 30. The controller 42 can acquire the optical image received by the objective optical system that is arranged in the distal-end rigid part 30.

As FIG. 2 shows, the living-tissue sampling instrument 14 includes a hollow tube 52, a sampling part 54, and a suction mechanism (suction unit) 56. The hollow tube 52 has flexibility. The sampling part 54 is provided at the distal end of the tube 52 and configured to sample living tissues. The suction mechanism 56 is arranged at the proximal end of the tube 52. The suction mechanism 56 includes a barrel (suction cavity) 62 and a plunger 64. The barrel 62 is the outer cylinder. The plunger 64 is the inner cylinder. The plunger 64 is fitted in the barrel 62, with its outer circumferential surface set in firm contacting with the inner circumferential surface of the barrel 62. The distal end of the barrel 62 connects the proximal end of the tube 52. The interior of the barrel 62 communicates with the interior of the tube 52. Thus, air is drawn from the tube 52 into the barrel (suction cavity) 62 when the operator pulls the plunger 64 from the barrel 62 toward him or her.

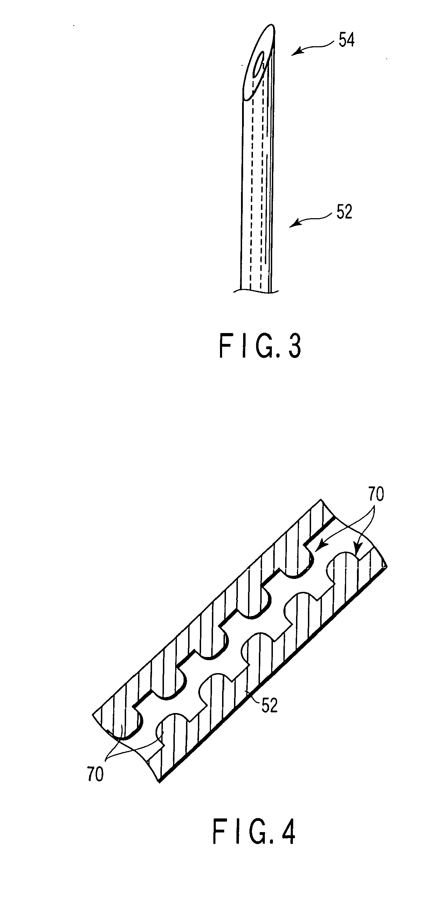

As shown in FIG. 3, the sampling part 54 is a hollow puncture needle. To sample tissues from a body cavity, the sampling part 54 may preferably be thrust into the body cavity to sample, thereby to sample tissues or the exfoliated tissues and cells contained in the liquids (e.g., liquid in the abdominal cavity, liquid in the thoracic cavity, pancreatic juice, bile, etc.).

The tube 52 and the sampling part 54 are formed almost integral with each other. That is, there is no distinct border between the tube 52 and the sampling part 54. The length of the tubular member composed of the tube 52 and sampling part 54 is much greater than the length of the instrument-insertion channel. The sampling part 54 has an outer diameter of, for example, 0.7 mm (equivalent to rated value 22G for an injection needle). The sampling part 54 and the tube 52 have the same maximum inner diameter of 0.3 mm. The material of the sampling part 54 and tube 52 is not particularly limited. Nonetheless, the material should be resistant to corrosion, because the sampling part 54 and tube 52 are inserted into patients. They should be made preferably of, for example, stainless steel, corrosion-resistant resin or the like. Stainless steel is particularly preferred as their material.

It is sufficient of the tube 52 to have at least one passage. Preferably, it may have projections 70 arranged in the passage at random positions, as is illustrated in FIG. 4. In this embodiment, the tube 52 has 48 projections 70, while the tubular member, i.e., the combination of the tube 52 and the sampling part 54, is, for example, 1550 mm long. The number of projections 70 provided may, of course, change in accordance with the length and diameter of the tubular member and with the length and shape of the projections 70.

The projections 70 are shaped like a sphere or a hemisphere formed by cutting a cocoon into halves by an appropriate plane. It is desirable that each projection 70 should have a height of, for example, about 0.15 mm, as measured from the inner surface, a major axis of about 0.5 mm and a minor axis of about 0.2 mm. If the tube 52 has many projections 70 as is shown, the projections 70 are positioned at random in the tube 52 and are not uniform in shape. Hence, the interior of the tube 52 is formed not uniform. The shape of the projections 70 is not limited to this. The projections 70 may have any shape so long as the tube 52 is clogged and any living tissue can be destroyed as it passes through the tube 52.

The projections 70 on the inner surface of the tube 53 can be formed by any method. Nonetheless, they may be formed by, for example, by sting the tube 52 from outside with a jig (not shown) shaped like a needle whose distal end has a specific shape. This method can process the tube 52 after a puncture needle (hollow needle) has been formed, as sampling part 54, at the distal end of the tube 52. This method is economically advantageous because the molding is easy to perform.

How the endoscope system 10 according to this embodiment is operated will be explained below.

The distal-end rigid part 30 of the insertion section 22 of the endoscope 12 is inserted into a body cavity, to a position near the living tissue that should be sampled. At this time, the endoscope 12 is rotated around the axis of the insertion section 22, thereby rotating the insertion section 22, and the angulation knob 36 on the operation section 24 of the endoscope 12 is operated, thereby bending the bending part 28 of the insertion section 22. Thus, the objective optical system provided in the distal-end rigid part 30 of the insertion section 22 receives the optical image of the living tissue to be sampled. The operator can therefore observe a desirable optical image of the living tissue at the controller 42.

Then, the living-tissue sampling instrument 14 is guided into the body cavity through the instrument-insertion channel from the forceps value 32 that is provided at the proximal end of the insertion section 22.

At this time, the forceps-raising base holds an appropriate part of the tube 52 of the living-tissue sampling instrument 14, at the distal end of the instrument-insertion channel. In this state, the insertion section 22 of the endoscope 12 is moved toward the living tissue, whereby the sampling part 54 stings the living tissue. Alternatively, the operator may hold the proximal end of the tube 52 of the living-tissue sampling instrument 14 and move the tube 52 forwards and backwards, thereby piercing the living tissue with the sampling part 54. In this case, the hebel does not hold the tube 52.

The plunger 64 of the suction mechanism 56 is pulled from the barrel 62, while the living tissue remains pierced with the sampling part 54. As a result, the living tissue is drawn by suction first through the sampling part 54 and then through the tube 52. As the living tissue passes from the distal end of the tube 52 toward the proximal end thereof, it collides many times with the projections 70 protruding from the inner surface of the tube 52 and is thereby destroyed. As the living tissue collides many times with the projections 70, it is decomposed into cells. The living tissue, thus destroyed, is retained in the interior (suction cavity) of the barrel 62 of the suction mechanism 56.

The living-tissue sampling instrument 14 is pulled from the living tissue in the body cavity, together with the insertion section 22 of the endoscope 12. The living tissue is no longer pierced with the sampling part 54 of the living-tissue sampling instrument 14.

The tube 52 of the living-tissue sampling instrument 14 may be released from the hebel, while the insertion section 22 of the endoscope 12 held immovable. In this case, the living-tissue sampling instrument 14 can be moved back and forth through the instrument-insertion channel. The sampling part 54 of the living-tissue sampling instrument 14 can thereby be pulled out of the living tissue.

The living-tissue sampling instrument 14 is pulled from the instrument-insertion channel, while the living tissues destroyed stays in the tube 52 or the barrel 62 of the suction mechanism 56. The tissue destroyed is then removed from the living-tissue sampling instrument 14 and transported to the examination room. In the examination room, the tissue is subjected to pathological examination, biochemical analysis, genomic analysis, or the like.

In view of the foregoing, the following can be said of the present embodiment.

Many projections 70 are provided on the inner surface of the tube 52 of the living-tissue sampling instrument 14. Therefore, the living tissue sampled collides many times with the projections 70 as it is drawn by suction. The tissue is finally decomposed into cells. This saves the time for destroying the living tissue in the examination room before nucleic acid is extracted in preparation for the pathological examination, biochemical analysis, genomic analysis, or the like. The time required for preparation for pathological examination, biochemical analysis, genomic analysis, or the like can be therefore shortened. No apparatus for making such preparation needs to be installed in the examination room. In addition, the endoscope system has a great economical advantageous because the tube 52 is easy to produce by molding.

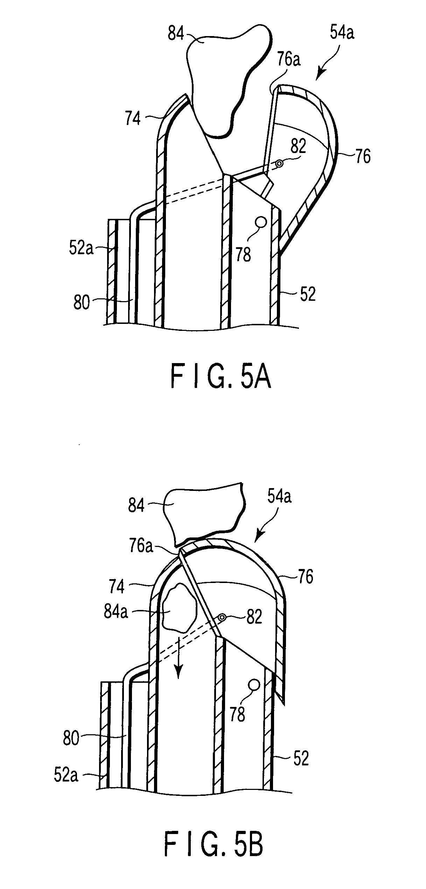

As described above, the sampling part 54 is a puncture needle in the present embodiment. Instead, the sampling part 54 may preferably have a forceps 54a as is shown in FIGS. 5A and 5B.

As FIGS. 5A and 5B show, the forceps 54a includes a fixed jaw 74 which is formed integral with the distal end of the tube 52 and a movable jaw 76. The movable jaw 76 can rotate around a pivotal pin 78, to open and close with respect to the fixed jaw 74.

A support member 82 is provided on the movable jaw 76. The support member 82 holds the distal end of a jaw-opening/closing wire 80. The opening/closing wire 80 extends in the hollow cylinder 52a that surrounds the tube 52, in axial alignment with the tube 52. The proximal end of the opening/closing wire 80 is provided in a wire-pulling/slackening mechanism (opening/closing mechanism, not shown). The wire-pulling/slackening mechanism extends parallel to the suction mechanism 56. Therefore, the movable jaw 76 is opened with respect to the fixed jaw 74 as the wire-pulling/slackening mechanism moves the opening/closing wire 80 toward the distal end of the tube 52. Conversely, the movable jaw 76 is closed with respect to the fixed jaw 74 as the wire-pulling/slackening mechanism moves the opening/closing wire 80 toward the proximal end of the tube 52.

As described above, the forceps 54a on the sampling part 54 of the living-tissue sampling instrument 14 is inserted through the instrument-insertion channel of the endoscope 12 and arranged near the target living tissue 84 that should be sampled. The operator may operate the wire-pulling/slackening mechanism, opening the movable jaw 76 with respect to the fixed jaw 74 of the forceps 54a. The distal end of the forceps 54a remaining in this state is thereby pushed onto the living tissue.

The wire-pulling/slackening mechanism is operated, closing the movable jaw 76 with respect to the fixed jaw 74. The living tissue 84 clamped between the blade 76a at the edge of the movable jaw 76 and the edge of the fixed jaw 74 is thereby cut. The living tissue 84a thus cut is dragged into the forceps 54a.

The suction mechanism 56 is operated in this state, drawing the living tissue by suction. Thus drawn, the living tissue collides many times with the projections 70 provided in the tube 52 and is thereby destroyed. The tissue is finally decomposed into cells.

The forceps 54a is of single-swing type, in which the movable jaw 76 can open and close with respect to the fixed jaw 74. The forceps 54a may be of double-swing type, instead. The blade 76a may be serrated or may have any other shape. The sampling part 54 may preferably be a forceps with a needle, or a combination of a forceps and a puncture needle. If this is the case, the forceps holds the target living tissue pierced with the puncture needle.

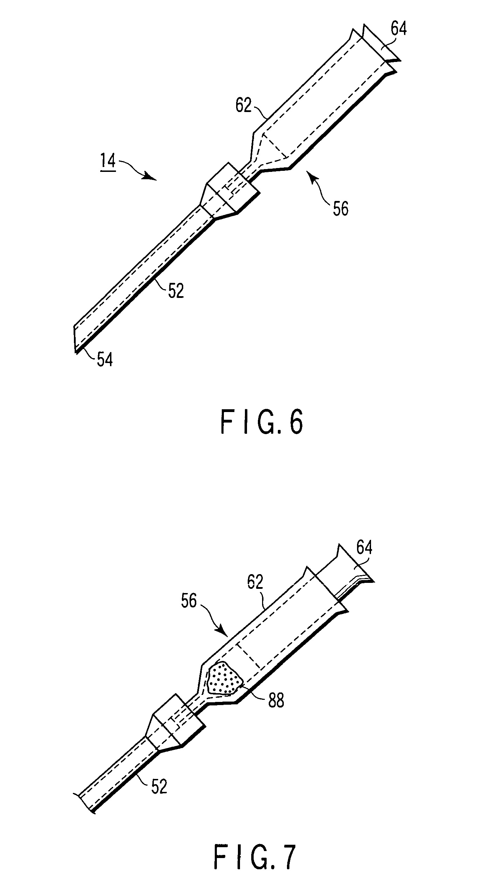

The second embodiment will be described with reference to FIG. 6. This embodiment is a modification of the first embodiment. The components identical to those described in conjunction with the first embodiment are designated by the same reference numerals and will not be described in detail.

FIG. 6 show a living-tissue sampling instrument 14 that is configured to sample living tissues in a percutaneous manner, without using the endoscope 12 shown in FIG. 1. This living-tissue sampling instrument 14 includes a rigid tube 52, a sampling part 54, and a suction mechanism 56. The sampling part 54 is provided at the distal end of the tube 52. The suction mechanism 56 is provided at the proximal end of the tube 52.

As shown in FIG. 6, the sampling part 54 is a hollow puncture needle. The tube 52 and the sampling part 54 are formed integral with each other. The sampling part 54 has an outer diameter of, for example, 0.7 mm (equivalent to rated value 22G for an injection needle). The sampling part 54 and the tube 52 have the same maximum inner diameter of 0.3 mm. The material of the sampling part 54 and tube 52 is not particularly limited. Nonetheless, the material should be resistant to corrosion. They should be made preferably of, for example, stainless steel, corrosion-resistant resin or the like. Stainless steel is particularly preferred as their material.

The tube 52 and the sampling part 54 are combined, providing a tubular member, which is, for example, 80 mm long. That is, the tubular member, i.e., the combination of the tube 52 and the sampling part 54, is much shorter than tubular member described in conjunction with the first embodiment.

On the inner surface of the tube 52, for example, 20 projections 70 are formed at random positions. These projections 70 are, for example, of the same type as those explained in conjunction with the first embodiment.

How the living-tissue sampling instrument 14 according to this embodiment is operated will be explained below.

The sampling part 54 of the living-tissue sampling instrument 14 is thrust into the living tissue in a percutaneous manner. In this state, the plunger 64 is pulled from the barrel 62. As a result, the living tissue is drawn by suction first through the puncture needle of the sampling part 54 and the through the tube 52. As the living tissue thus sampled passes through the tube 52, it collides many times with the projections 70 protruding from the inner surface of the tube 52 and is thereby destroyed. As the living tissue collides many times with the projections 70, it is decomposed into cells. The living tissue, thus destroyed, is retained in the tube 52 and the barrel 62 of the suction mechanism 56.

The sampling part 54 of the living-tissue sampling instrument 14 is pulled out of the living tissue. The living tissue destroyed by suction is removed from the living-tissue sampling instrument 14 and transported to the examination room. In the examination room, the tissue is subjected to pathological examination, biochemical analysis, genomic analysis, or the like.

In view of the foregoing, the following can be said of the present embodiment.

Many projections 70 are provided on the inner surface of the tube 52 of the living-tissue sampling instrument 14. Therefore, the living tissue sampled collides many times with the projections 70 as it is drawn by suction. The tissue is finally decomposed into cells. This saves the time for destroying the living tissue in the examination room before nucleic acid is extracted in preparation for the pathological examination, biochemical analysis, genomic analysis, or the like. Thus, the same advantage can be attained as in the first embodiment, though the endoscope 12 (see FIG. 1) is not used in sampling the living tissue in a percutaneous manner.

In the present embodiment, a hollow sampling tube can be used as sampling part 54, in place of the hollow puncture needle. The sampling tube may have the same shape as, for example, the sampling part 54 shown in FIGS. 3 and 6. The outer diameter of this sampling tube is not particularly limited. Nonetheless, the tube has an outer diameter of about 1 mm to 10 mm and an inner diameter of about 0.5 mm to 8 mm in most cases. Preferably, the sampling tube is made of flexible plastics or rubber. Such a sampling tube is used, inserted into a duct in the patient (e.g., pancreatic duct, bile duct, etc.). The secretory liquid is sampled from the duct and passed through a filter (not shown), which filters out the exfoliated tissue or cells. The tissue or cells thus sampled can be subjected to an examination. This embodiment does not use the endoscope 12. Nevertheless, it is desirable to guide the living-tissue sampling instrument 14, which has a hollow sampling tube used as sampling part 54, through the instrument-insertion channel of the endoscope 12 and then to insert the instrument 14 into the duct in the patient.

The third embodiment will be described with reference to FIG. 7. This embodiment is a modification of the living-tissue sampling instrument 14 according to the first and second embodiments. The components identical to those described in conjunction with the first and second embodiments are designated by the same reference numerals and will not be described in detail. If the tube 52 is longer than the insertion section 22 of the endoscope 12 and has flexibility, it can be used together with the endoscope 12 in the endoscope system 10. If the tube 52 is hard and rigid, the living-tissue sampling instrument 14 can be used alone in a percutaneous manner. This holds true of the fourth to eleventh embodiments that will be described later.

As FIG. 7 shows, the barrel 62 of the suction mechanism 56 of the living-tissue sampling instrument 14 according to the present embodiment contains a stabilizing agent 88 (preservative solution) that prevents, for example, deterioration of RNA.

The living tissue drawn into and decomposed in the living-tissue sampling instrument 14 is mixed with the stabilizing agent 88 in the barrel 62. The living tissue decomposed can therefore remain stable.

In view of the foregoing, the following can be said of the present embodiment.

The living tissue sampled is destroyed in situ and decomposed into cells. The cells can be stabilized with the stabilizing agent 88. This saves the time for destroying the living tissue in the examination room before nucleic acid is extracted.

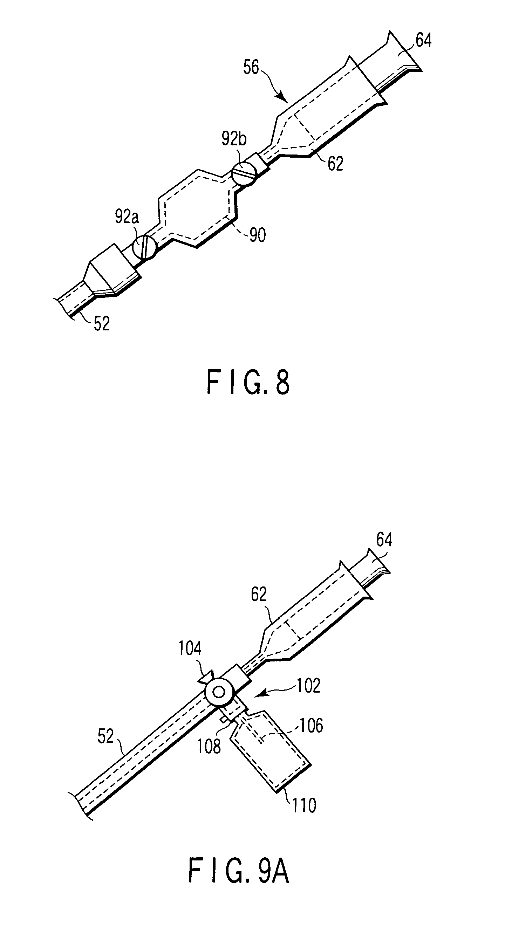

The fourth embodiment will be described with reference to FIG. 8. This embodiment is a modification of the living-tissue sampling instrument 14 according to the first to third embodiments.

As FIG. 8 shows, a container 90 is provided between the proximal end of the tube 52 and the barrel 62 of the suction mechanism 56. The container 90 has connector parts 92a and 92b, which are connected to the proximal end of the tube 52 and the barrel 62 of the suction mechanism 56, respectively. The container may contain air only or may contain stabilizing agent 88 (preservative solution) that prevents, for example, deterioration of RNA.

The living tissue drawn by the suction mechanism 56 and then decomposed is stored in the container 90. After the tissue has been drawn into the container 90, the connector parts 92a and 92b are disconnected from the tube 52 and the suction mechanism 56. The connector parts 92a and 92b are then plugged. The container 90 is transported.

The container 90 can therefore transported with ease. That is, the living tissue decomposed can be easily transported. Once the container 90 has been so disconnected, the living tissue can be easily taken out.

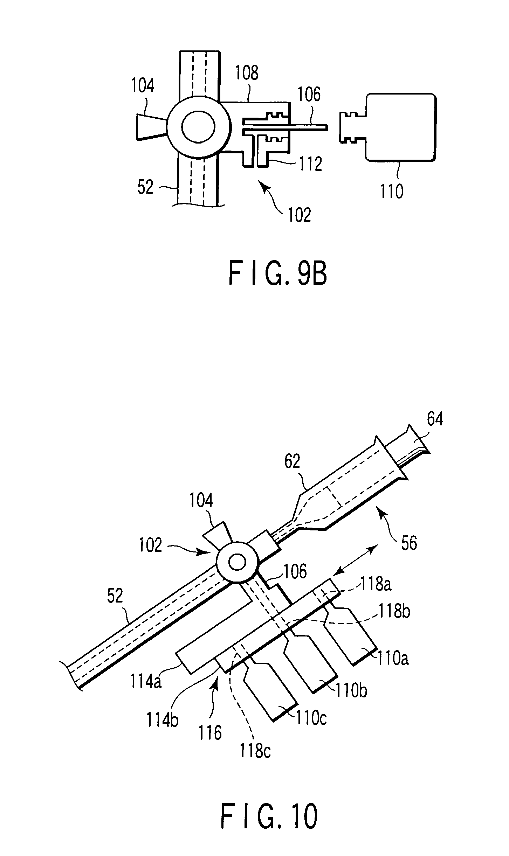

The fifth embodiment will be described with reference to FIGS. 9A and 9B. This embodiment is a modification of the living-tissue sampling instrument 14 according to the first to fourth embodiments.

As FIG. 9A shows, a valve mechanism (passage-switching valve) 102 is arranged at the proximal end of the tube 52. The valve mechanism 102 has an opening/closing knob 104. When operated, the opening/closing knob 104 is able to be switched between a communicating state in which to communicate the tube 52 to the barrel 62 so that the living tissue may be drawn in, and a cutoff state in which to cut off the tube 52 from the barrel 62 so that the living tissue can no longer be drawn in. The valve mechanism 102 has a housing 108 that has a passage 106 that communicates with the barrel 62 while the tube 62 remains disconnected from the barrel 62.

A container 110 can be removably attached to the housing 108. The housing 108 has a pressure-releasing port 112, which communicates with the interior of the container 110 as long as the container 110 remains attached to the housing 108.

How the living-tissue sampling instrument 14 according to this embodiment is operated will be explained below.

The opening/closing knob 104 of the valve mechanism 102 is operated, making the tube 52 communicates with the barrel 62. The living tissue is thereby drawn in. At this point, the living tissue drawn is temporarily stored in the barrel 62.

After the tissue has been thus drawn, the opening/closing knob 104 of the valve mechanism 102 is operated and switched to the cutoff state setting the tube 52 out of communication with the barrel 62. In this cutoff state, the plunger 64 is pushed into the barrel 62. As a result, the living tissue is expelled from the barrel 62. The living tissue is then moved into the container 110 through the passage 106 of the valve mechanism 102. Thus, the living tissue, already decomposed into cell, is thus stored in the interior of the container 110. Although the living tissue is expelled from the passage 106 as the plunger 64 is pushed into the barrel 62, the pressure-releasing port 112 prevents the pressure in the container 110 from rising with respect to the outside pressure.

After the living tissue has been stored into the container 110, the container 110 is detached from the housing 108 of the valve mechanism 102. A cover (not shown) is put on the container 110. The container 110 is transported so that the living tissue may be subjected pathological examination, biochemical analysis, genomic analysis, or the like.

The use of the valve mechanism 102 renders it easy to store the decomposed living tissue into the container 110. In addition, the container 110 is easy to transport.

The sixth embodiment will be described with reference to FIG. 10. This embodiment is a modification of the living-tissue sampling instrument 14 according to the fifth embodiment.

As FIG. 10 shows, the valve mechanism 102 includes a first sliding housing 114a. A cartridge mechanism (passage-switching mechanism) 116 is provided on the first sliding housing 114a. The cartridge mechanism 116 has a second sliding housing 114b. The second sliding housing 114b has first, second and third communication paths 118a, 118b and 118c. The communication paths 118a, 118b and 118c can communicate with the above-mentioned passage 106 as the second sliding housing 114b slides on the first sliding housing 114a. That is, the second sliding housing 114b can slide with respect to the first sliding housing 114a. Three containers 110a, 110b and 110c can be removably connected to the communication paths 118a, 118b and 118c, respectively.

How the living-tissue sampling instrument 14 according to this embodiment is operated will be explained below.

The second sliding housing 114b of the cartridge mechanism 116 is mounted on the first sliding housing 114a of the valve mechanism 102, so that the housing 114b may slide on the first sliding housing 114a. The containers 110a, 110b and 110c are connected to the communication paths 118a, 118b and 118c of the second sliding housing 114b, respectively.

The opening/closing knob 104 of the valve mechanism 102 is operated and switched to the communicating state setting the tube 52 into communication with the barrel 62. The sampling part 54 is thrust into the target living tissue. The suction mechanism 56 is used, drawing the living tissue into the barrel 62. The sampling part 54 is pulled out of the living tissue.

Then, the second sliding housing 114b of the cartridge mechanism 116 is slid with respect to the first sliding housing 114a of the valve mechanism 102. As a result, the communication path 118a communicates with the passage 106.

The opening/closing knob 104 of the valve mechanism 102 is then operated and switched to the cutoff state setting the tube 52 out of communication with the barrel 62. The plunger 64 is pushed into the barrel 62. As a result, the living tissue is expelled from the barrel 62. The living tissue is thereby moved, partly or entirely, from the barrel 62 into the container 110a through the passage 106 of the valve mechanism 102 and the first communication path 118a. At this time, all living tissue stored in the barrel 62 should better be stored into the container 110a through the first communication path 118a.

The sampling part 54 is thrust into the living tissue again. The opening/closing knob 104 of the valve mechanism 102 is operated, setting the tube 52 into communication with the barrel 62. Then, the suction mechanism 56 is used, drawing the living tissue into the barrel 62. The sampling part 54 is the pulled from the living tissue.

The second sliding housing 114b of the cartridge mechanism 116 is slid with respect to the first sliding housing 114a of the valve mechanism 102. The second communication path 118b is thereby set into communication with the passage 106. The opening/closing knob 104 of the valve mechanism 102 is then operated and switched to the cutoff state setting the tube 52 out of communication with the barrel 62. In this cutoff state, the plunger 64 is pushed into the barrel 62. The living tissue is thereby expelled from the barrel 62 and stored into the container 110b through the passage 106 of the valve mechanism 102 and the second communication path 118b of the cartridge mechanism 116. At this time, part of the living tissue is left in the barrel 62.

The second sliding housing 114b of the cartridge mechanism 116 is slid with respect to the first sliding housing 114a of the valve mechanism 102. The third communication path 118c is thereby set into communication with the passage 106. In this state, the plunger 64 is further pushed into the barrel 62. The living tissue is thereby expelled from the barrel 62 and stored into the container 110c through the passage 106 of the valve mechanism 102 and the third communication path 118c of the cartridge mechanism 116. The living tissue is thus accumulated in the container 110c.

The containers 110a, 110b and 110c are removed from the second sliding housing 114b. Covers (not shown) are put on the containers 110a, 110b and 110c, respectively. The containers are then transported.

Thus, the containers 110a, 110b and 110c contain different parts of the living tissues. In other words, the three continuous parts of the living tissue are now stored in the containers 110a, 110b and 110c, respectively.

The living tissue now stored in the containers 110b and 110c have been sampled at the same site in the patient. To analyze the same living tissue by different methods, the living tissue stored in the barrel 62 can be distributed to the containers 110b and 110c. While being so distributed, the tissue can be protected against contamination. This helps to make the analyses more accurate than otherwise.

The containers 110a, 110b and 110c may contain different reagents (not shown), different stabilizing agents (not shown), or the like, respectively.

In view of the foregoing, the following can be said of the present embodiment.

Living tissues sampled at various sites in the patient may be continuously stored, one after another, into the containers 110a, 110b and 110c, respectively.

In order to analyze the same living tissue by different methods, the tissue temporarily accumulated in the suction mechanism 56 may be distributed to the containers 110a, 110b and 110c. While being so distributed, the tissue can be protected against contamination. This helps to make the analyses more accurate than otherwise.

As described above, the second sliding housing 114b of the cartridge mechanism 116 is slid with respect to the first sliding housing 114a of the valve mechanism 102 in the present embodiment. Instead, both housings 114a and 114b may be of rotating type. Further, the number of paths made in the second sliding housing 114b need not be limited to three. Rather, the housing 114b may have more or fewer paths, in accordance with the type of analysis which the living tissue will undergo.

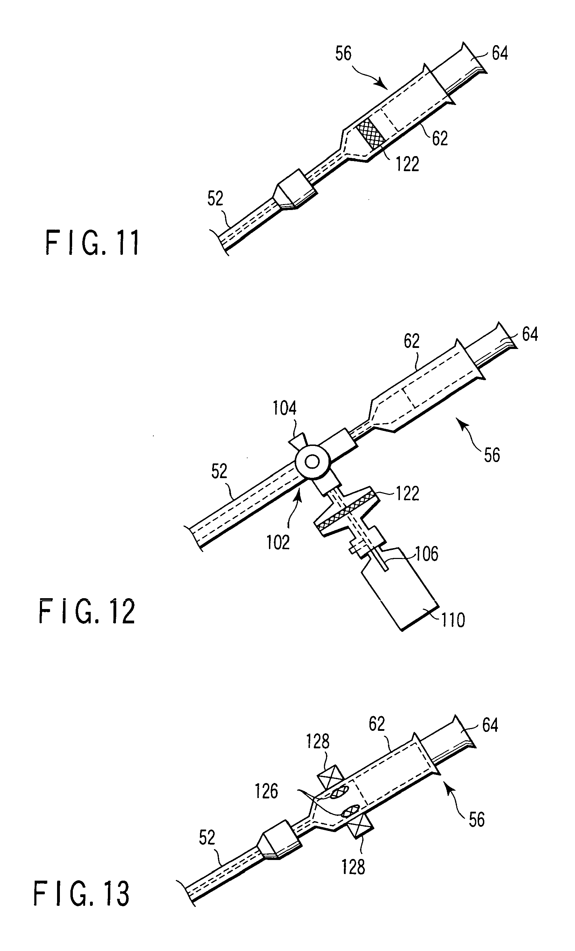

The seventh embodiment will be described with reference to FIG. 11. This embodiment is a modification of the living-tissue sampling instrument 14 according to the first embodiment.

As FIG. 11 shows, the barrel 62 of the suction mechanism 56 contains a filter 122 that catches a part of the living tissue drawn into the barrel 62. The filter 122 has bio-compatibility compound to living tissues, on the side opposed to the tube 52. In this embodiment, this bio-compatibility compound is, for example, a cancer-cell capturing antibody that has a specific behavior to cancer cells. That is, the cancer-cell capturing antibody is applied to the filter 122.

The living tissue sampled by the sampling part 54 into the tube 52 and destroyed to cells as explained in conjunction with the first embodiment passes through the filter 122 provided in the barrel 62. The filter 122 catches the cancer cells only.

After the living tissue has been drawn into the barrel 62, the filter 122 is removed from the barrel 62. At this time, only the cancer cells can be easily removed, because nothing but the cancer cells is captured on the filter 122.

As described above, the filter 122 is arranged in the barrel 62 of the suction mechanism 56 in the present embodiment. Instead, the filer 122 may be arranged in the tube 52.

The eighth embodiment will be described with reference to FIG. 12. This embodiment is a modification of the living-tissue sampling instrument 14 according to the fifth to seventh embodiments.

As FIG. 12 shows, a filter 122 is provided in the housing 108 that has the passage 106 of the valve mechanism 102. The filter 122 is arranged, blocking the passage 106 to capture a part of the living tissue being drawn into the passage 106.

Thus, after the living tissue has been temporarily accumulated in the barrel of the suction mechanism 56, the opening/closing knob 104 of the valve mechanism 102 is operated and switched to the cutoff state setting the tube 52 out of communication with the barrel 62, as has been described in conjunction with the fifth embodiment. The living tissue is thereby expelled. At this point, the filter 122 captures the cancer cells only. The other part of the living tissue is accumulated in the container 110.

After the living tissue has been expelled from the barrel 62 into the container 110, the filter 122 is taken out of the housing 108 of the valve mechanism 102. At this time, the filter 122 retains the cancer cells only. Hence, only the cancer cells can be removed.

The ninth embodiment will be described with reference to FIG. 13. This embodiment is a modification of the living-tissue sampling instrument 14 according to the seventh and eighth embodiments.

As FIG. 13 shows, magnetic beads 126 are mounted on the inner surface of the barrel 62 of the suction mechanism 56. The beads 126 hold an antibody that has a specific behavior to cancer cells. A permanent magnet 128 is arranged outside the barrel 62 and surrounds the magnetic beads 126. If a living tissue exists in the barrel 62 when the suction mechanism 56 is operated to drawn by suction, cancer cells, if any, in the tissue will be coupled to the magnetic beads 126. The magnetic beads 126 now holding the cancer cells are collected at a prescribed position. Only the cancer cells can therefore be taken out.

The present embodiment uses a permanent magnet 128. The permanent magnet 128 may be replaced by an electromagnet. The electromagnet is more easy to use, because it generates a magnetic field when supplied with an electric current.

Though not shown herein, the magnetic beads 126 or the magnet 128 may preferably be arranged in the passage 106 of the valve mechanism 102 or the container 110, in the fourth to sixth embodiment and the eighth embodiment.

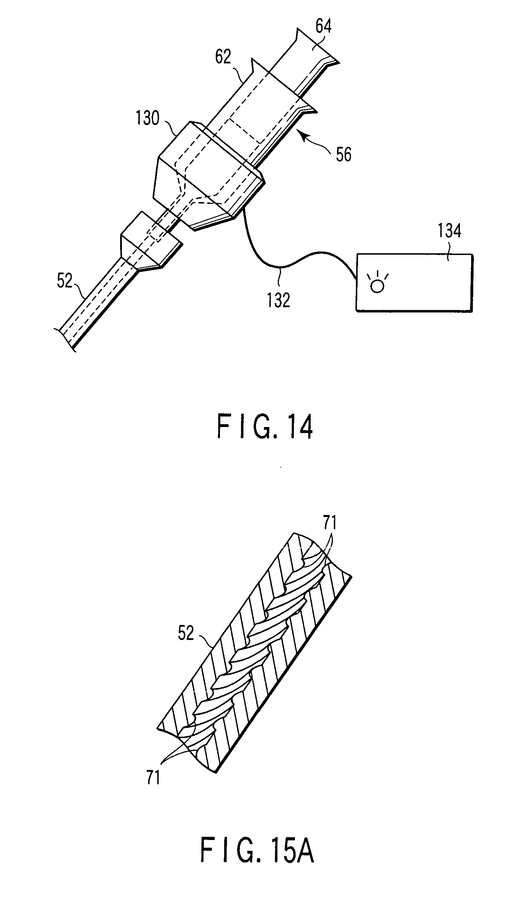

The tenth embodiment will be described with reference to FIG. 14. This embodiment is a modification of the living-tissue sampling instrument 14 according to the first embodiment.

As FIG. 14 shows, a cooling mechanism 130 is provided outside the barrel 62 of the suction mechanism 56 and surrounds the barrel 62. A cable 132 connects a controller 134 to the cooling mechanism 130. The method how the cooling mechanism 130 performs cooling is not limited. Nonetheless, it is desirable to use an electronic device, such as a Peltier element, because such a device is small.

The controller 134 controls the temperature of the cooling mechanism 130. So controlled in terms of temperature, the cooling mechanism 130 may, for example, freeze the living tissue sampled into the barrel 62, drawn by suction, by means of the suction mechanism 56. Thus, the living tissue destroyed and decomposed into cells at the position where it has been sampled is frozen and, hence, stabilized. The tissue cells are thereby prevented from deteriorating. Further, the time for destroying the living tissue in the examination room before nucleic acid is extracted can be saved.

In the present embodiment, the cooling mechanism 130 is provided outside the barrel 62 of the suction mechanism 56 and surrounds the barrel 62. Instead, the cooling mechanism 130 may be arranged, surrounding the container 90, 110, 110a, 110b or 110c in, for example, the fourth, fifth, sixth or eighth embodiment.

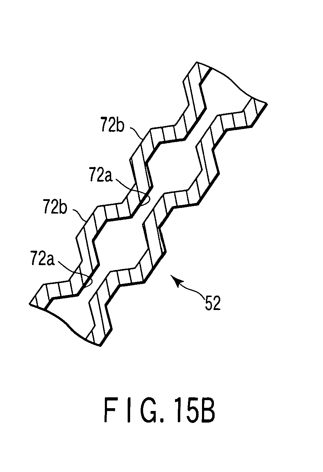

The eleventh embodiment will be described with reference to FIGS. 15A and 15B. This embodiment is a modification of the living-tissue sampling instrument 14 according to the first embodiment.

As FIG. 15A shows, the tube 52 has a continuous projection 71 in its inner surface. The projection 71 defines, for example, a female screw.

As FIG.. 15B shows, the tube 52 has been squeezed at several parts, forming projections 72a. The other parts not squeezed define depressions 72b. Hence, the tube 52 has an inner diameter that continuously changes. The projections 72a may have the same height or different heights, by changing the condition of squeezing the parts of the tube 52. The projections 72a of the tube 52 may be made in state of random position and random size.

Several embodiments have been described in detail, with reference to the drawings. Nevertheless, the present invention is not limited to the embodiments described above. The present invention includes any embodiments that can be achieved without departing the scope and spirit of the invention.

### Industrial Applicability

The present invention can provide a living-tissue sampling instrument, an endoscope system, and a method of sampling living tissues, which can easily and quickly destroy sampled living tissues, thereby to shorten the time required to prepare for pathological examination, biochemical analysis, genomic analysis, or the like.

## Claims

1. A living-tissue sampling instrument **characterized by** comprising:
a hollow tubular member which has a distal end portion having a sampling part to sample living tissue, and a distal end portion having a suction mechanism configured to draw living tissue sampled by the sampling part,
wherein the tubular member has projections extending inwards, on least one part of an inner circumferential surface.

2. The living-tissue sampling instrument according to claim 1, **characterized in that** the projections are provided at random in the tubular member.

3. The living-tissue sampling instrument according to claim 1 or 2, **characterized in that** the tubular member includes an inner diameter that is unevenness because of the projections.

4. The living-tissue sampling instrument according to any one of claims 1 to 3, **characterized in that** the projections are formed independently of one another.

5. The living-tissue sampling instrument according to any one of claims 1 to 3, **characterized in that** at least some of the projections are continuous to one another.

6. The living-tissue sampling instrument according to any one of claims 1 to 5, **characterized in that** the projections have been formed by applying a pressure on the tubular member from outside.

7. The living-tissue sampling instrument according to any one of claims 1 to 6, **characterized in that** the suction mechanism includes a suction cavity which is able to accumulate the living tissue sampled by the sampling part.

8. The living-tissue sampling instrument according to claim 7, **characterized in that** a solution to be mixed with the living tissue sampled by the sampling part is accumulated in the suction cavity.

9. The living-tissue sampling instrument according to claim 7 or 8, **characterized in that** a container capable of storing the living tissue sampled by the sampling part is removably provided between a proximal end of the tubular member and the suction cavity.

10. The living-tissue sampling instrument according to claim 9, **characterized in that** the container accumulates a solution to be mixed with the living tissue sampled by the sampling part.

11. The living-tissue sampling instrument according to claim 7 or 8, **characterized in that** the suction mechanism includes a valve mechanism provided between a proximal end of the tubular member and the suction cavity, the valve mechanism being able to be switched between a communicating state in which to communicate the sampling part to the suction cavity, and a cutoff state in which to cut off the sampling part to the suction cavity, the valve mechanism including a passage that communicates with the suction cavity in the cutoff state.

12. The living-tissue sampling instrument according to claim 11, **characterized in that** a container capable of storing the living tissue sampled by the sampling part is arranged at an end of the passage of the valve mechanism.

13. The living-tissue sampling instrument according to claim 11, **characterized in that** a passage-switching mechanism includes a plurality of communication passages to be selectively connected to the passage, and is arranged to being able to connect and disconnect containers capable of storing the living tissue to and from the communication passages, respectively.

14. The living-tissue sampling instrument according to any one of claims 1 to 13, **characterized in that** at least one of the tubular member and the suction mechanism includes a filter configured to capture a part of the living tissue sampled by the sampling part.

15. The living-tissue sampling instrument according to claim 14, **characterized in that** the filter includes a bio-compatibility compound to living tissues on one side facing the sampling part.

16. The living-tissue sampling instrument according to any one of claims 1 to 13, **characterized in that** at least one of the tubular member and the suction mechanism includes:
magnetic beads each having, on outer surface, bio-compatibility compound to the living tissues; and
one of a magnet and an electromagnet, which applies magnetic force to the magnetic beads.

17. The living-tissue sampling instrument according to claim 15 or 16, **characterized in that** the bio-compatibility compound to the living tissues is a cancer-cell recognizing antibody.

18. The living-tissue sampling instrument according to any one of claims 1 to 17, **characterized in that** a cooling mechanism configured to cool the living tissue sampled by the sampling part is connected to the suction mechanism.

19. The living-tissue sampling instrument according to any one of claims 1 to 18, **characterized in that** the sampling part includes a hollow puncture needle to be thrust into living tissues.

20. The living-tissue sampling instrument according to claim 19, **characterized in that** the tubular member is rigid at an outer circumferential surface.

21. The living-tissue sampling instrument according to claim 19, **characterized in that** the tubular member includes flexibility.

22. The living-tissue sampling instrument according to any one of claims 1 to 19, **characterized in that** the sampling part includes a forceps able to be opened and closed to hold living tissues.

23. The living-tissue sampling instrument according to claim 22, **characterized in that** the tubular member includes flexibility.

24. An endoscope system **characterized by** comprising:
an endoscope including:
an elongated insertion section which has an instrument-insertion channel extending in an axial direction; and
an operation section which is arranged at a proximal end of the insertion section; and
a living-tissue sampling instrument including:
a hollow tubular member having such an outer diameter that the member is inserted into and pulled from the instrument-insertion channel, having projections extending inwards, on least one part of an inner circumferential surface, and being longer than the instrument-insertion channel;
a sampling part provided at a distal end of the tubular member, able of be inserted and configured to sample living tissues; and
a suction mechanism provided at a proximal end of the tubular member and configured to draw, by suction, a living tissue sampled by the sampling part.

25. The endoscope system according to claim 24, **characterized in that** the projections are provided at random in the tubular member.

26. The endoscope system according to claim 24 or 25, **characterized in that** the tubular member has an inner diameter that is unevenness because of the projections.

27. The endoscope system according to any one of claims 24 to 26, **characterized in that** the projections are formed independently of one another.

28. The endoscope system according to any one of claims 24 to 26, **characterized in that** at least some of the projections are continuous to one another.

29. The endoscope system according to any one of claims 24 to 28, **characterized in that** a solution to be mixed with the living tissue sampled by the sampling part is accumulated in the suction mechanism.

30. The endoscope system according to claim 29, **characterized in that** the suction mechanism includes a container capable of storing the living tissue sampled by the sampling part and accumulate the solution, and a suction part able to be switched between a communicating state in which to communicate with the container and a cutoff state in which to be disconnected from the container.

31. The endoscope system according to any one of claims 24 to 29, **characterized in that** the suction mechanism includes:
a suction cavity which is able to accumulate the living tissue sampled by the sampling part; and
a valve mechanism which is provided between a proximal end of the tubular member and the suction cavity, the valve mechanism being able to be switched between a communicating state in which to communicate the sampling part to the suction cavity, and a cutoff state in which to cut off the sampling part to the suction cavity, the valve mechanism including a passage that communicates with the suction cavity in the cutoff state.

32. The endoscope system according to any one of claims 24 to 31, **characterized in that** the sampling part includes a hollow puncture needle to be thrust into living tissues.

33. The endoscope system according to any one of claims 24 to 31, **characterized in that** the sampling part includes a tube which is able to be inserted into a duct in a living body.

34. The endoscope system according to any one of claims 24 to 31, **characterized in that** the sampling part includes a forceps able to be opened and closed to hold living tissues, and an opening/closing mechanism is provided at a proximal end of the tubular member and is configured to open and close the forceps.

35. A method of sampling living tissues, by using a living-tissue sampling instrument which includes:
a hollow tubular member having a sampling part for sampling a living tissue at a distal end portion of the tubular member, and a suction mechanism for drawing by suction a living tissue sampled by the sampling part at a proximal end portion of the tubular member; and
projections extending inwards of the tubular member, on least one part of an inner circumferential surface, said method **characterized by** comprising:
drawing the living tissue sampled by the sampling part and making the living tissue collide with the projections provided in the tubular member, thereby destroying the living tissue; and
accumulating, in the suction mechanism, the living tissue made to collide with the projections and thereby destroyed.

36. A method of sampling living tissues, by using a living-tissue sampling instrument which includes:
a hollow tubular member having a sampling part for sampling a living tissue at a distal end portion of the tubular member, and a suction mechanism for drawing by suction a living tissue sampled by the sampling part at a proximal end portion of the tubular member;
projections extending inwards of the tubular member on least one part of an inner circumferential surface; and
a container capable of storing the living tissue sampled by the sampling part and provided between a proximal end of the tubular member and the suction mechanism, said method **characterized by** comprising:
drawing the living tissue sampled by the sampling part and making the living tissue collide with the projections provided in the tubular member, thereby destroying the living tissue;
accumulating, in the container, the living tissue destroyed; and
removing the container from a junction between the proximal end of the tubular member and the suction mechanism.

37. A method of sampling living tissues, by using a living-tissue sampling instrument which includes:
a hollow tubular member having a sampling part for sampling a living tissue at a distal end portion of the tubular member, and a suction mechanism for drawing by suction a living tissue sampled by the sampling part at a proximal end portion of the tubular member;
projections extending inwards of the tubular member on least one part of an inner circumferential surface;
a container capable of storing the living tissue sampled by the sampling part and provided between a proximal end of the tubular member and the suction mechanism; and
a valve mechanism provided between a proximal end of the tubular member and the suction mechanism, the valve mechanism being able to be switched between a communicating state in which to communicate the sampling part to the suction mechanism, and a cutoff state in which to cut off the sampling part to the suction mechanism, the valve mechanism including a passage that communicates with the suction mechanism in the cutoff state, said method **characterized by** comprising:
drawing the living tissue sampled by the sampling part and making the living tissue collide with the projections provided in the tubular member, thereby destroying the living tissue;
temporarily accumulating, in the container, the living tissue destroyed;
switching the valve mechanism, thereby setting the suction mechanism and an interior of the container in the communicating state;
expelling the living tissue from the suction mechanism into the container through the valve mechanism; and
detaching the container.

38. The method of sampling living tissues, according to any one of claims 35 to 37, **characterized in that** the drawing the living tissue sampled by the sampling part and making the living tissue collide with the projections provided in the tubular member, thereby destroying the living tissue includes projecting the sampling part from a distal end of a instrument-insertion channel of an endoscope.
